# EUROPEAN PATENT APPLICATION

(11) **EP 1 500 428 A1**
(43) Date of publication of application: **26.01.2005**
(21) Application number: 03746469.0
(22) Date of filing: 14.04.2003
(51) Int. Cl.: B01J 19/08, A61N 5/06, F02M 27/04, C02F 1/68, C02F 1/30

(54) **ENERGY RADIATION DEVICE AND ENERGY IRRADIATION APPARATUS COMPRISING THE SAME**

(30) Priority: 17.04.2002 JP 2002115404
(71) Applicant: Kusuikou Co., Ltd., Tokyo 103-0002 (JP)
(72) Inventor: Saitou, Hidehiko, Tokyo 136-0076 (JP)
(74) Representative: Grünecker, Kinkeldey, Stockmair & Schwanhäusser Anwaltssozietät
(86) International application number: PCT/JP2003/004675
(87) International publication number: WO 2003/086616

(57) **Abstract**

An energy radiation device which comprises a special ore layer (14) comprising a mixture of tourmaline and an anion ore such as a ceramic and, laminated on the special ore layer (14), a colored material layer (16) comprising a specifically colored material. The colored material layer (16) may be formed by laminating a plurality of layers, and an intermediate layer (18) containing gold, silver, titanium or carbon may be provided between the special ore layer (14) and the colored material layer (16) or between two layers constituting the colored material layer (16). The energy radiation device allows the utilization of the effectiveness of tourmaline with good efficiency and with ease and simplicity.

## Description

### Technical Field

The present invention relates to an energy radiation device and an irradiation apparatus comprising the same, and more particularly to an energy radiation device which utilizes vibrational energy such as electromagnetic wave, and anion radiated from tourmaline and the like.

### Background Technology

Heretofore, it has been known that vibrational energy such as electromagnetic wave having various wavelengths and anion is radiated from tourmaline, and a variety of benefits is derived from such radiation of vibrational energy of tourmaline.

More specifically, it has been known that tourmaline has various benefits such as preventive effects of coagulation of or adherence to blood vessel of cholesterol in a living body, effects for adjusting body fluid to be weakly alkaline, effects for generating far infrared rays, mineralization of water, weak-alkalization of water, activation of water, washing effects, effects for hardly staining a bathtub, washbasin and the like, deodorizing effects, effects for removing foul odor, antibacterial effects, and effects for maintaining freshness.

For this reason, it has been desired to provide a manner for capable of utilizing efficiently and easily such benefits of tourmaline.

The present invention has been made in view of the above-described desirable needs, and an object of the present invention is to provide an energy radiation device and an energy irradiation apparatus comprising the same by which benefits of tourmaline can be efficiently and easily utilized.

### Disclosure of the Invention

In order to achieve the above-described object, an energy radiation device according to the present invention comprises a special ore layer prepared by mixing tourmaline with an anion ore; and a colored material layer made of a colored material having a predetermined color and which is laminated on the above-described special ore layer.

Furthermore, an energy radiation device according to the present invention comprises a special ore layer prepared by mixing tourmaline with an anion ore; and plural colored material layers each made of a colored material having a predetermined color and which are laminated on the above-described special ore layer.

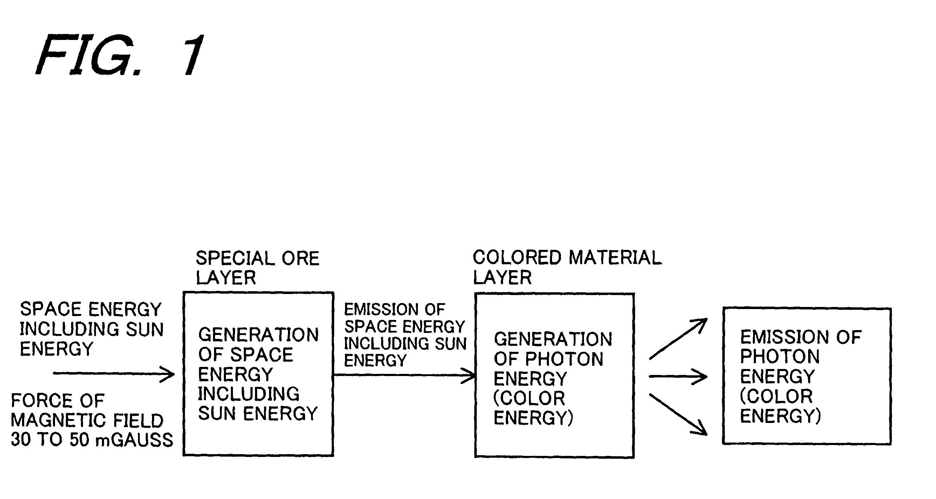

According to the above-described present invention, when vibrational energy such as space energy including sun energy, in other words, vibrational energy such as electromagnetic wave having a wavelength zone of space energy including sun energy is received, a vibrational energy such as a very weak electromagnetic wave or an anion having a wavelength (wavelengths in a wavelength zone extending from far infrared rays to x-ray) in response to a color of a colored material layer (an expression "vibrational energy such as a very weak electromagnetic wave or an anion having a wavelength in response to a color of a colored material layer" is hereinafter referred optionally to as "photon energy" or "color energy" in the present specification) is radiated in accordance with functions of tourmaline and an anion ore composed of a special ore layer generating efficiently space energy including sun energy as vibrational energy as well as a color of a colored material layer constituting a colored material layer (see FIG. 1).

It is to be noted that photon energy emits very weak energy with respect to all the substances, the energy is very weak, but it has a mass of a high density, and has a very weak vibrational energy.

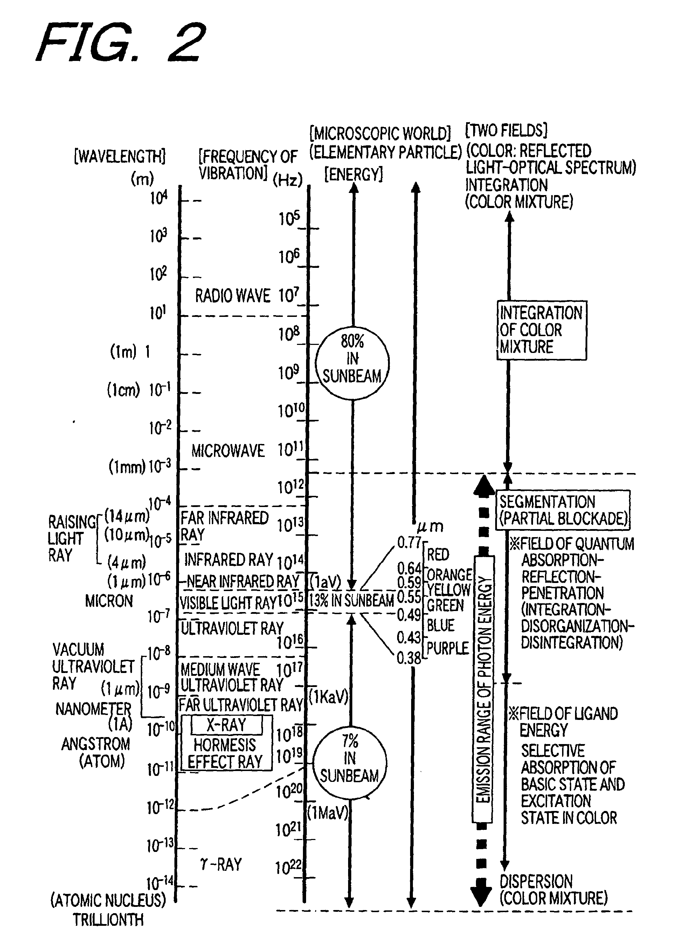

When an explanation will be continued by referring to a diagram of FIG. 2 indicating categories between photon energy and electromagnetic wave (wavelength of photon = field of quantum and field of ligand energy), photon energy reaches microscopic world and acts upon trillionth (atomic nucleus). This is because there is a coalescence of absorption, reflection, penetration in quantum field; and selective absorption of a ground state and an excitation state of a color in a field of ligand energy.

And, the above-described plural colored material layers may be prepared from colored materials having different colors one another.

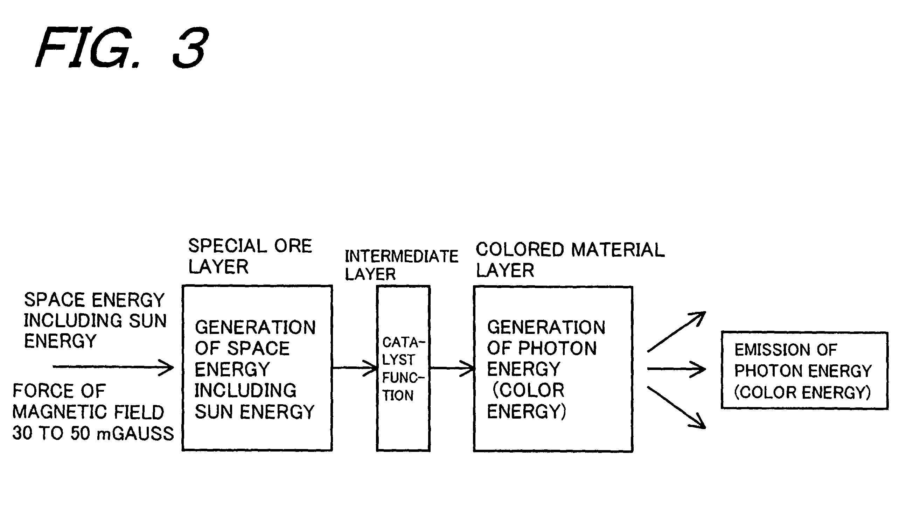

Furthermore, either an intermediate layer containing gold, silver, titanium, carbon or the like may be provided between the above-described special ore layer and the above-described colored material layer (see FIG. 3), or an intermediate layer containing gold, silver, titanium, carbon or the like may be provided between any one of regions defined between the above-described plural colored material layers.

As described above, when the intermediate layer containing gold, silver, titanium, carbon or the like is provided, the intermediate layer functions as a catalyst, so that color energy radiated from the energy radiation device according to the present invention is amplified.

Moreover, when a color of a colored material layer is adjusted, a wavelength of color energy radiated from the colored material layer can be changed, whereby the wavelength of color energy can be fitted to a use application of a target to which the color energy is to be irradiated.

Furthermore, an energy irradiation apparatus provided with the energy radiation device according to the present invention comprises a hollow body a part of or the whole walls of which are constituted by the energy radiation device or devices of the present invention; and a space energy radiation device disposed in the interior of the above-described hollow body.

In the energy irradiation apparatus provided with the energy radiation device, either a copper sheet may be disposed on a part of the above-described walls, or an aluminum sheet may be disposed on a part of the above-described walls.

In the case where a copper sheet is disposed on a part of the above-described walls, the outer side of the above-described copper sheet is covered with an aluminum sheet.

In addition, the above-described space energy radiation device may be constituted by the use of a symmetrical reverse winding coil or an asymmetrical reverse winding coil; or it may be constituted so as an NS magnet to repel an SN magnet.

### Brief Description of the Drawings

FIG. 1 is an explanatory diagram showing functions of an energy radiation device according to the present invention.
FIG. 2 is an explanatory diagram showing categories and the like between photon energy and electromagnetic wave.
FIG. 3 is an explanatory diagram showing functions of an energy radiation device according to the present invention.
FIG. 4(a) is a conceptual, constructional, explanatory view illustrating an example of an embodiment of an energy radiation device according to the present invention, and FIGS. 4(b) and (c) are conceptual, constructional, explanatory vies each illustrating a third modification.
FIGS. 5(a), (b) and (c) are conceptual, constructional, explanatory views each illustrating a fourth modification wherein FIG. 5(a) corresponds to FIG. 4(a), FIG. 5(b) corresponds to FIG. 4(b), and FIG. 5(c) corresponds to FIG. 4(c).
FIG. 6 is a diagram for explaining relationships between wavelength zones of color energy and emotion-five solid organs.
FIG. 7 is an explanatory diagram illustrating relationships between influence of specific colors-specific glands and wavelengths of photon energy.
FIG. 8 is an explanatory diagram indicating a route through which color energy is recognized.
FIG. 9 is an explanatory diagram indicating functions of photon energy with respect to a target.
FIG. 10 is an explanatory diagram showing a hydrogen bond of water molecules.
FIG. 11 is an explanatory diagram showing clusters of water molecules.
FIG. 12 is an explanatory diagram showing a situation wherein an energy radiation device is mounted on an intake air line positioned between a motor vehicle engine and an air cleaner.
FIG. 13 is a report of a driving experiment for a motor vehicle wherein an energy radiation device is mounted on an intake air line.
FIG. 14 is a report of a driving experiment for a motor vehicle wherein an energy radiation device is mounted on an intake air line.
FIG. 15 is a report of a driving experiment for a motor vehicle wherein an energy radiation device is mounted on an intake air line.
FIG. 16 is a report of a driving experiment for a motor vehicle wherein an energy radiation device is mounted on an intake air line.
FIG. 17 is a report of a driving experiment for a motor vehicle wherein an energy radiation device is mounted on an intake air line.
FIG. 18 is a report of a driving experiment for a motor vehicle wherein an energy radiation device is mounted on an intake air line.
FIG. 19 is an explanatory diagram showing a situation wherein an energy radiation device is mounted on an intake air line positioned between a motor vehicle engine and an air cleaner.
FIG. 20 is an explanatory view indicating harmonization melody of sounds by means of color energy.
FIG. 21 is a diagram showing a table of effects in materials used in every targets of color energy.
FIGS. 22(a) and (b) are views each showing an example of an energy irradiation apparatus comprising an energy radiation device according to an embodiment of the present invention wherein (a) is a perspective view a part of which is cut away, and (b) is a sectional view taken along the line A-A of (a).
FIGS. 23 (a) and (b) are explanatory diagrams each showing a symmetrical reverse winding coil as a space energy radiation device wherein (a) indicates a case of applying AC, while (b) indicates a case of applying DC.
FIGS. 24 (a) and (b) are explanatory diagrams each showing an asymmetrical reverse winding coil as a space energy radiation device wherein (a) indicates a case of applying AC, while (b) indicates a case of applying DC.
FIG. 25 is an explanatory view showing a construction of a space energy radiation device wherein NS and SN magnets are disposed so as to repel one another.

### Explanation of Reference Numerals

- 10: ENERGY RADIATION DEVICE
- 12: SUBSTRATE
- 14: SPECIAL ORE LAYER
- 16: COLORED MATERIAL LAYER
- 18: INTERMEDIATE LAYER
- 100: ENGINE
- 102: AIR CLEANER
- 104: INTAKE AIR LINE
- 106: REFLECTION PLATE
- 108: REFLECTION PLATE
- 200: ENERGY IRRADIATION APPARATUS COMPRISING ENERGY RADIATION DEVICE
- 202: BOTTOM
- 204: SIDE WALL
- 206: COPPER SHEET
- 208: ALUMINUM PLATE
- 210: SPACE ENERGY RADIATION DEVICE
- 300: SYMMETRICAL REVERSE WINDING COIL
- 400: ASYMMETRICAL REVERSE WINDING COIL
- 500: NS MAGNET
- 600: SN MAGNET
- E: COLOR ENERGY

### Best Mode for Embodying the Invention

In the following, an example of embodiments of the energy radiation device and the energy irradiation apparatus comprising the same according to the present invention will be described in detail by referring to the accompanying drawings.

In the following description, the same or equivalent components are designated by the same reference characters, respectively, and overlapped descriptions are to be omitted.

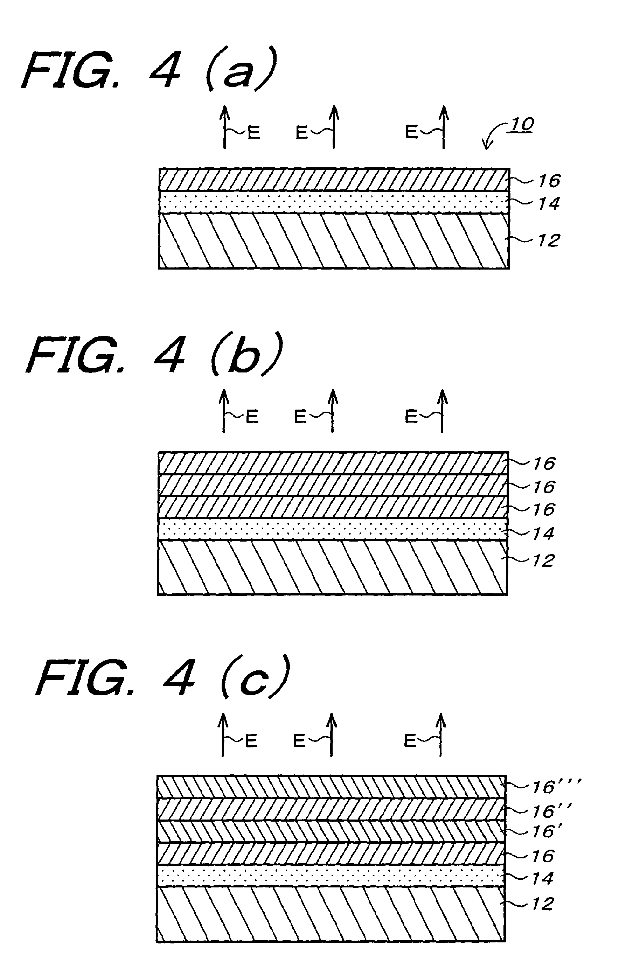

First, FIG. 4(a) shows a conceptual, constructional, explanatory view illustrating an example of an embodiment of the energy radiation device according to the present invention.

The energy radiation device 10 is obtained by forming a special ore layer 14 on a substrate 12, and then forming a colored material layer 16 on the special ore layer 14.

In this case, the special ore layer 14 is a layer formed from an admixture consisting of tourmaline and an anion ore such as ceramics. A mixing ratio of tourmaline and an anion ore may be an equal amount to each other. However, it is not necessarily required that both the components are in an equal amount to each other, but their mixing ratio may be properly changed in such that an amount of either component is made more than that of the other in response to a variety of applications. For instance, as mentioned hereinafter, it is preferred that a higher amount of an anion ore than that of tourmaline is used in the case where color energy radiated from the energy radiation device 10 is irradiated onto water for the sake of mineralization of water, weak alkalization of water, or activation of water. On one hand, it is preferred that a higher amount of tourmaline than that of an anion ore is used in the case where color energy radiated from the energy radiation device 10 is irradiated onto air sucked into a motor car engine for the sake of purification of exhaust gas from such motor car engine.

Furthermore, it is desirable that tourmaline ore and anion ores to be used are such a mixture prepared by admixing ores produced in respective areas positioned in four cardinal points of north, south, east and west regions with centering on an area where the energy radiation device 10 is used. It is to be noted that the above-described respective areas of four cardinal points are not only specified by those in Japan, but also those in a global point of view. Namely, when an area where the energy radiation device 10 is employed is Japan, tourmaline ore and anion ores produced in an east area, for example, west cost district in North America, in a west area, e.g. a Southern Europe district, in a south area, e.g. Oceania district, and in a north area, e.g. Siberia district may be used.

In case of forming the special ore layer 14 from tourmaline and an anion ore, first, tourmaline and the anion ore are powdered to obtain grains. Then, these grains are admixed in a proper mixing ratio, and thereafter they are sintered at a high temperature (for example, 860°C to 1250°C) to solidify the admixture, whereby the special ore layer is formed.

On one hand, the colored material layer 16 is a layer made of a colored material of a proper color (the term "colored material" in the present specification means a medium to which a predetermined color is given. In this case, for example, a proper material such as a colored paper, and a colored plastic sheet may be used as the colored material.

A color of a colored material constituting the colored material layer 16 in the energy radiation device 10 is a unitary color, but the colored material layer 16 may be composed of a colored material which is formed in which a plurality of colors exists mixedly.

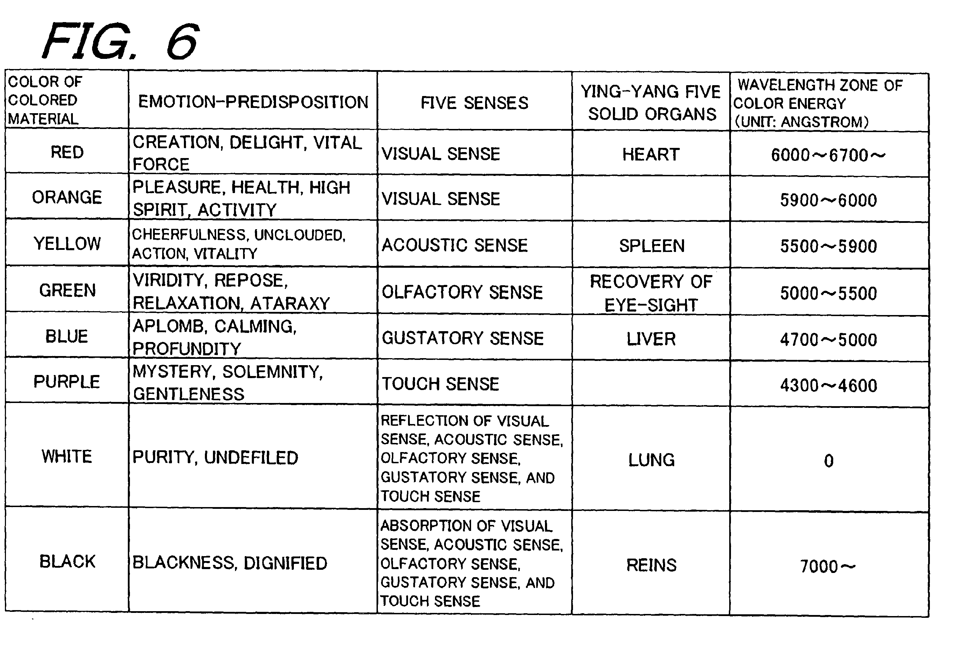

Furthermore, a variety of colors such as red, orange, yellow, green, blue, indigo, purple, white, and black may be properly used as a color of such colored material. Due to color energy radiated from the energy radiation device 10, efficacy in response to a color of a colored material constituting the colored material layer 16 can be obtained. The efficacy obtained by color energy radiated from the energy radiation device 10 which is derived from that in response to a color of a colored material constituting the colored material layer 16 will be described in detail hereunder by referring to FIG. 6 and the like.

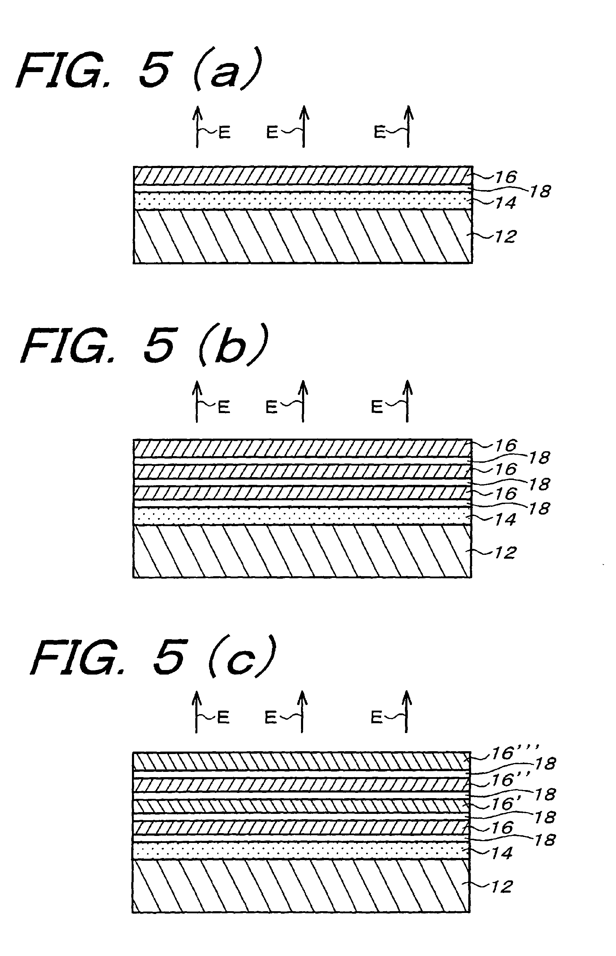

In the above construction, when vibrational energy such as space energy including sun energy, in other words, vibrational energy such as electromagnetic energy being in a wavelength zone of space energy including sun energy is received from the top surface of the energy radiation device 10, color energy being vibrational energy of very weak electromagnetic wave, anion ion or the like having a wavelength (a wavelength of a wavelength zone extending from far infrared rays to x-ray) in response to a color of a colored material is radiated in accordance with functions of tourmaline and an anion ore constituting the special ore layer 14 which generates efficiently space energy including sun energy as vibrational energy as well as a color of a colored material constituting the colored material layer 16. In FIGS. 4 (a) , (b) , and (c) and FIGS. 5 (a) , (b) , and (c), radiation of color energy is indicated by a reference character E.

Thus, when color energy radiated from the energy radiation device 10 is irradiated onto a target, various advantageous effects can be obtained. More specifically, when a target is a plant, the plant can be activated. When a target is an animal, activation of such living body can be attained. Moreover, it becomes possible to achieve atomization of chemical substances, mineralization of water, weak-alkalization of water or activation of water, purification of exhaust gas in a motor car engine.

In FIG. 6, relationships between wavelength zones of color energy and emotion-five solid organs are indicated. More specifically, the wavelength zones of color energy in response to colors of colored materials constituting the colored material layer 14, and targets to be activated by color energy in the case where the targets to which such color energy is irradiated are shown, respectively.

As shown in FIG. 6, when color energy radiated from the energy radiation device 10 is irradiated onto a human body in the case where, for example, a color of a colored material constituting the colored material layer 16 is red, creativity, delight, and vital force can be promoted, visual sense can be activated, and action of the heart can be activated.

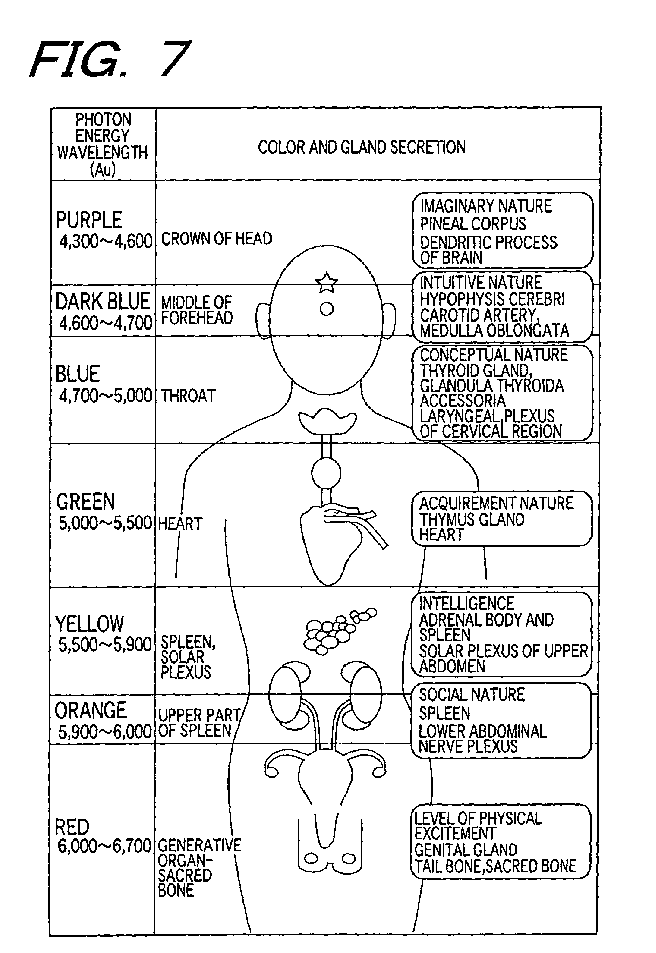

Furthermore, FIG. 7 is a diagram illustrating relationships between influence of specific colors-specific glands and wavelengths of photon energy. As shown in FIG. 7, a body of human being is classified by colors and gland secretion, so that the human body is composed of rainbow colors (seven colors) from the top of head to sacred bone. When color energy is irradiated onto respective parts in each color, cells can be activated.

Incidentally, it is acknowledged that elements of respective colored materials constituting the colored material layer 16 include, for example, oxygen, hydrogen, carbon, lithium, calcium, barium, manganese, iron, chromium, nickel, copper, zinc, strontium, cadmium, cobalt, manganese, aluminum, titanium, rubidium and the like, and each of them is in a kind of foison or nutrition.

Respective colored materials constituting the colored material layer 16 become perceived as colors, when they are reflected, and in this case, respective colored materials are reflected with respective wavelengths (a wavelength region of 4300 angstrom or more). On one hand, all the materials to be a target such as human body, and animals radiate also energy.

In these circumstances, when vibrational energy generated by the special ore layer 14 passes through the colored material layer 16, color energy is radiated in response to a color of a colored material constituting the colored material layer 16. Moreover, when wave energy generated by the special ore layer 14 passes through the colored material layer 16, compensation energy can be increased.

It is to be noted that all the materials of organic and inorganic substances perceive electromagnetic waves in a whole frequency region. This means that all creation reacts to electromagnetism of light and a color constituting light. For instance, a plant requires light for effecting photosynthesis. Such plant utilizes light energy through leaves, and it combines carbon dioxide taken out from air with water and microelements soaked up from earth to produce ingredients required for own growth and bleeding. Light energy of the sun is indispensable for synthesizing nutriments of plants.

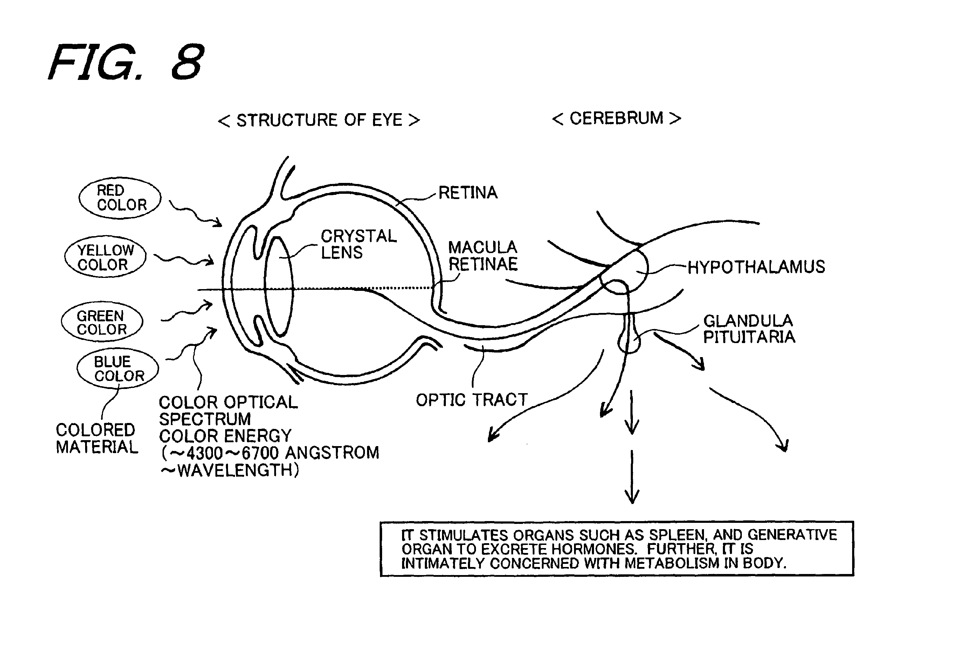

When light energy of the sun is applied to animals, a body of them is influenced directly. Namely, a human tissue receives chemical and physical changes. More specifically, as shown in a route for recognizing color energy of FIG. 8, when a human being or an animal catches color energy through its eyes, it simulates amphiblestrode to transfer the result to cells through hypothalamus, thereby stimulating electrons, molecules, and atomic nuclei.

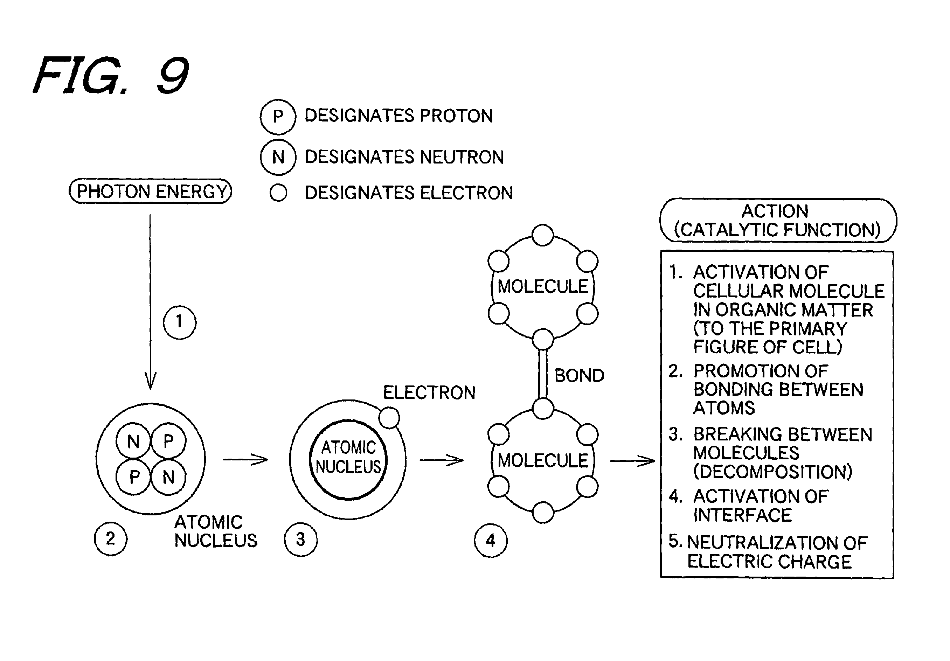

Moreover, as shown in a diagram of FIG. 9 illustrating functions of photon energy upon a target, a human being and an animal can receive also photon energy through its skin, so that the photon energy is transferred to cells to stimulate their molecules and atomic nuclei as in the case where photon energy is captured through its eyes.

Namely, photon energy exerts directly protons ((1) in FIG. 9) to change spin motions of the protons ((2) in FIG. 9). As a result, a rotation of an electron changes, so that physical properties of an atom change ( (3) in FIG. 9). Besides, a bonding situation of intermolecular atoms varies, and further bonds between molecules change also ((4) in FIG. 9).

Thus, the following functions appear.
1. Activation of cell molecules of an organic substance (It returns them to the original form of cells)
2. Promotion of interatomic bonds
3. Interatomic scission (decomposition)
4. Activation of interface
5. Neutralization of electric charge

For this reason, it may be considered that when color energy radiated from the energy radiation device 10 is irradiated onto a target, functions for returning impaired cells of the target to their original state appear. For instance, when the color energy radiated from the energy radiation device 10 is irradiated onto a target, it can be intended to increase anion ions, to improve bloodstream, to elevate immune strength, to maintain fine health, to prevent and recover disease, and to elevate powers of concentration.

In the following, influences of color energy exerting upon targets as a result of irradiating the color energy radiated from the energy radiation device 10 onto a target will be specifically described.

(1) When the color energy radiated from the energy radiation device 10 is irradiated onto a plant, basic tissue cells of the plant can be activated, whereby improvements in pest resistance, and improvements in absorptive power for fertilizer and manure are intended, so that the plant can revert to its proper state.

(2) When the color energy radiated from the energy radiation device 10 is irradiated onto a food, it is intended to promote freshness maintenance or fermentation of the food, resulting in improvements in storable duration, quality and productivity of the food.

(3) Water functions as a medium for conveying nutrition or energy to a body, cells, or a tissue. Accordingly, when the color energy radiated from the energy radiation device 10 is irradiated onto water and a human being or an animal drinks the water onto which the color energy has been irradiated (hereinafter referred optionally to "color energy water"), a viscosity in blood of the human being or the animal decreases, so that circulation of the blood becomes smooth to act on weakened cells and the vicinity thereof, resulting in acceleration of activation for the cells.

In addition, when color energy water is compared with usual water, a deuterium oxide concentration of the former water decreases as much as by about 30 ppm. Thus, to drink such color energy water brings about prevention of multiplication of cancer cells, besides it results in necrosis of such cancer cells. In this respect, a detailed description will be made hereinafter.

First, water is classified into light water and heavy water (According to another classification, water is classified into hard water, permanent hard water, temporary hard water, distilled water, and pure water).

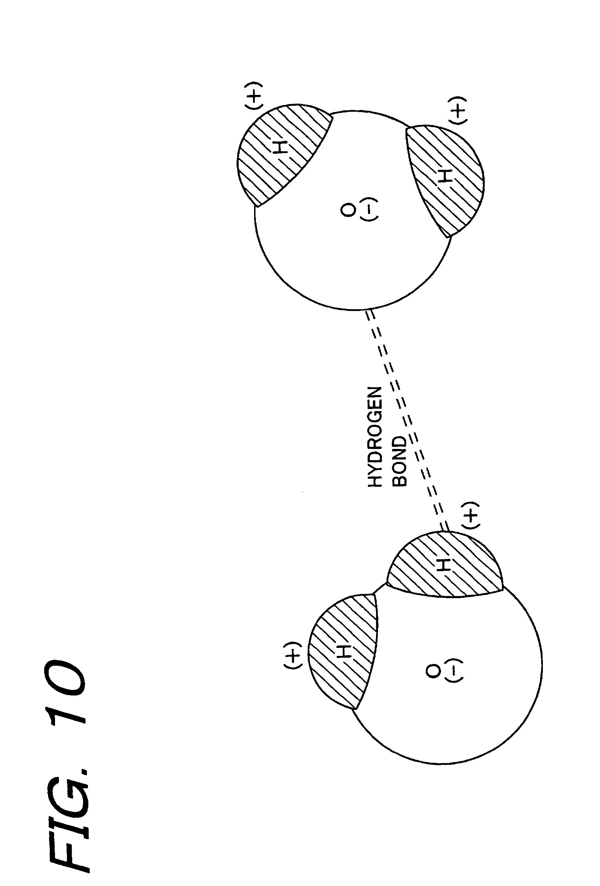

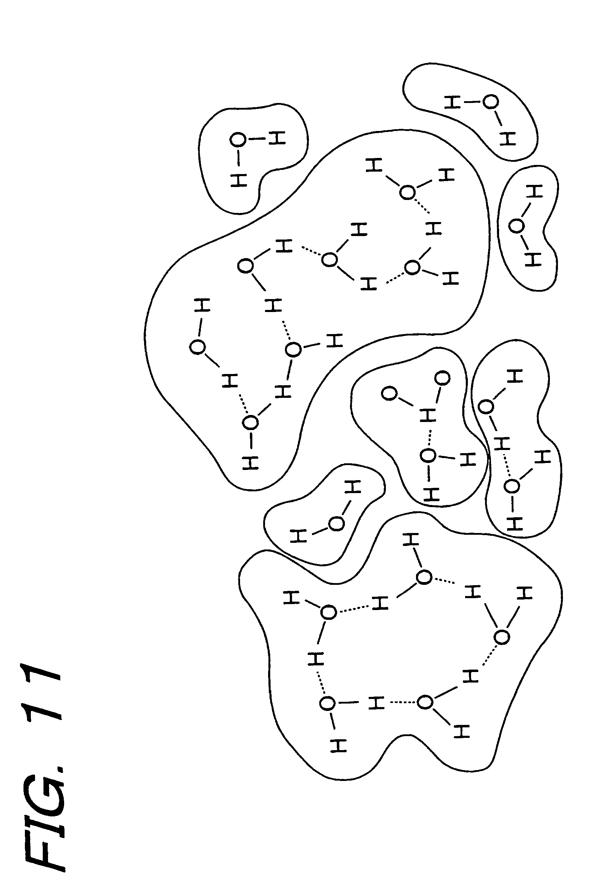

In general, a water molecule has a covalent bonding wherein two hydrogen atoms and one oxygen atom provide their electrons for the covalent bonding. In this respect, there is not only one water molecule, but also a number of water molecules to maintain their volumes thereby forming water. Moreover, water molecules move momentarily to repeat covalent bonding (see FIG. 10) , and they coalesce and loosen to form groups called by the name of cluster (see FIG. 11).

Namely, water exists not only in a single molecular form, but also in a cluster form which is constituted through hydrogen bonds, and it is said that water containing small clusters has nice taste.

Generally, water means a water molecule of atomic mass number of 18 composed of two hydrogen atoms each having 1 atomic mass number and one oxygen atoms having 16 atomic mass number, and such water is called by the name of light water.

Meanwhile, natural water (light water = usual water) on earth contains 150 water molecules of heavy water which has more than 18 atomic mass number per million water molecules. Such "existence of heavy water in light water" was found by Urey in 1932. Thereafter, it was found by Hungarian Dr. Somlyai (Doctor of Science, molecular biology academian) that light water from which heavy hydrogen deuterium is reduced necrotized cancer cells. In an article entitled "Let's defeat cancer!" by Dr. Somlyai, it is pointed out that significant changes appear in cells by reduction of only 140 heavy hydrogen deuterium per million (140 ppm) in a heavy water concentration of light water.

According to experiments of the inventor of the present application, a heavy water concentration of color energy water is only 121 heavy hydrogen deuterium per million (121 ppm) , so that a heavy water concentration is reduced by about 30 ppm as compared with that of usual water.

This means that effects for taking color energy water are in prevention of multiplication of cancer cells, and necrosis thereof. In addition, it may be concluded that drinking such color energy water results in health enhancement, and recovery of immunity in human body, whereby people can live always in a healthy body.

A reason for unsuitable application of heavy water as drinking water is in that it obstructs actions of oxygen (a high-molecular substance acting as a catalyst in a living body) , so that physiology is remarkably damaged.

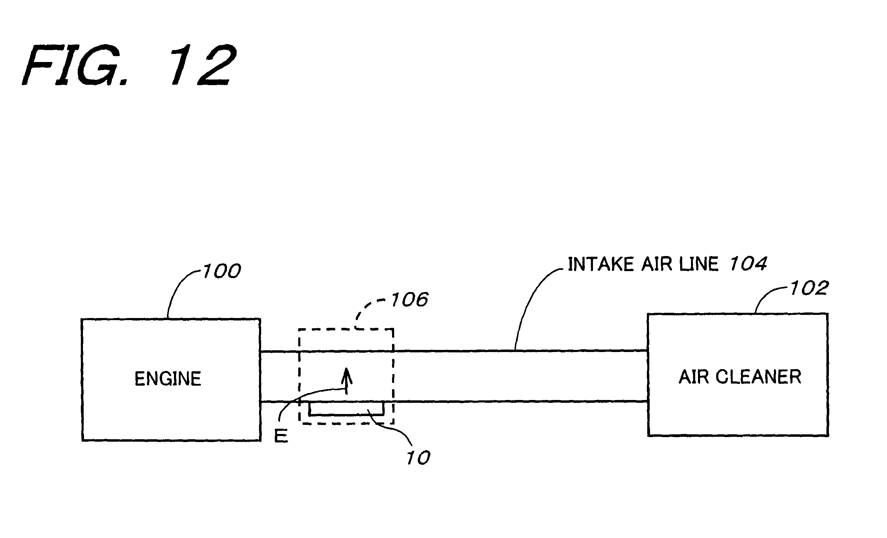

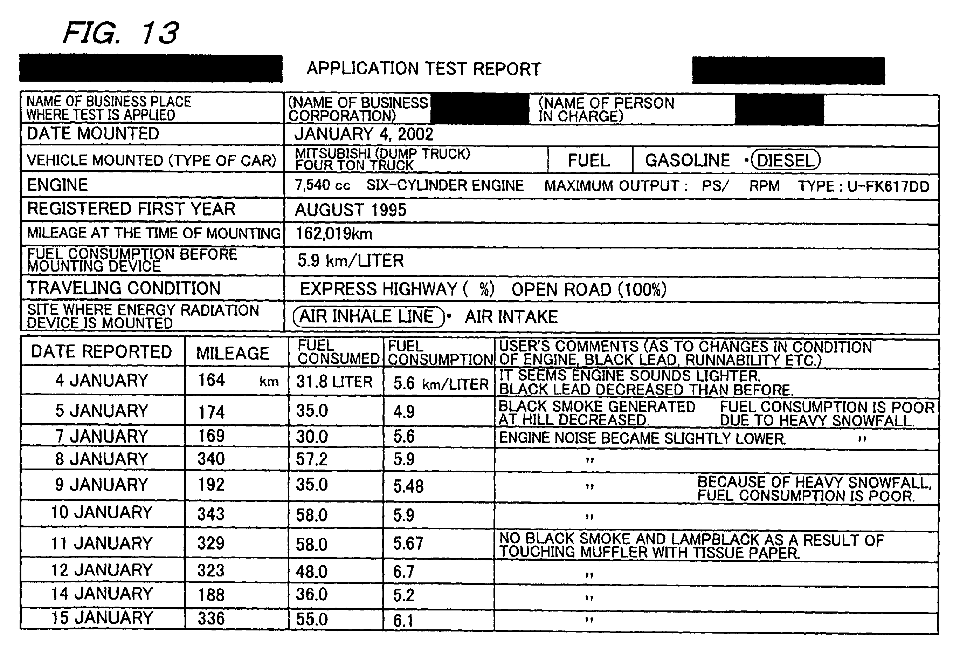

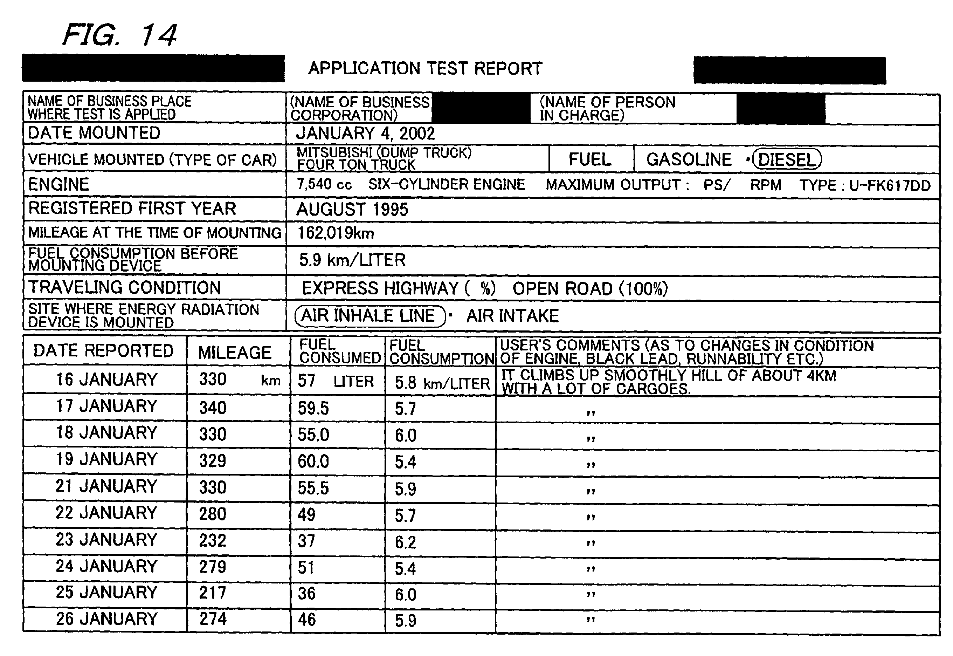

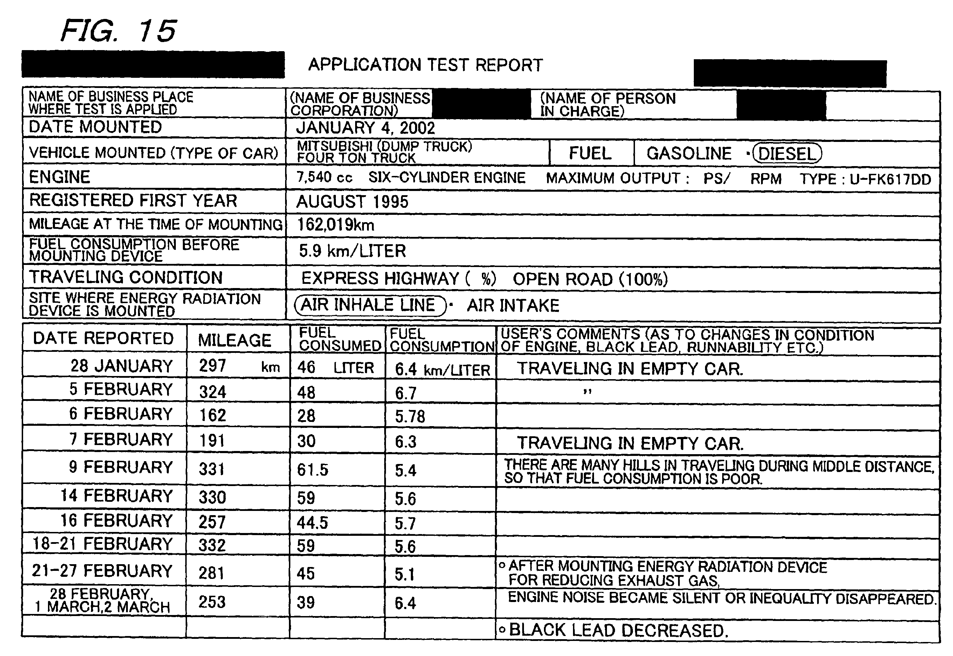

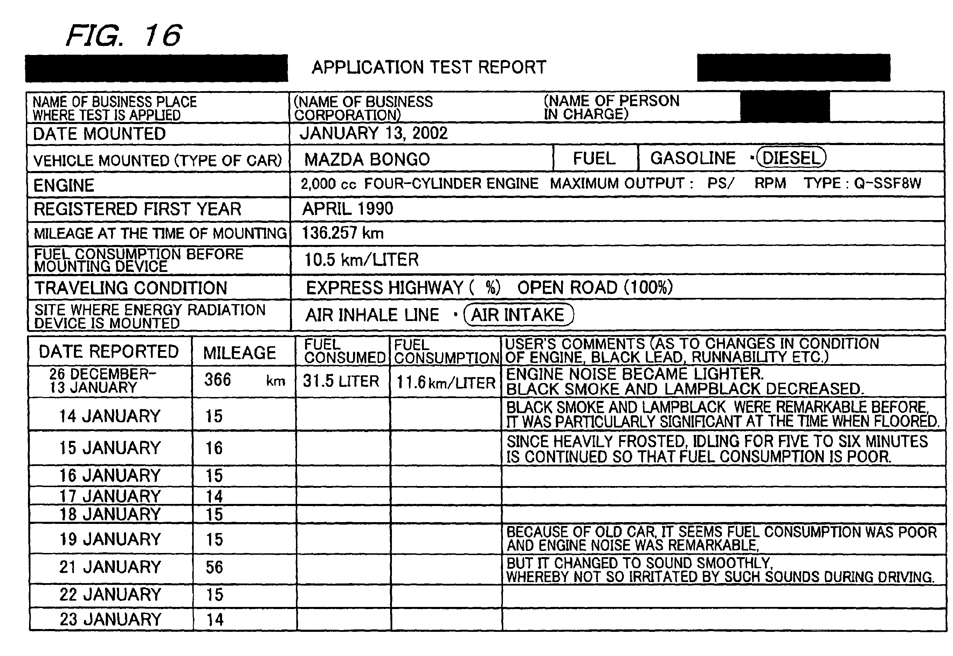

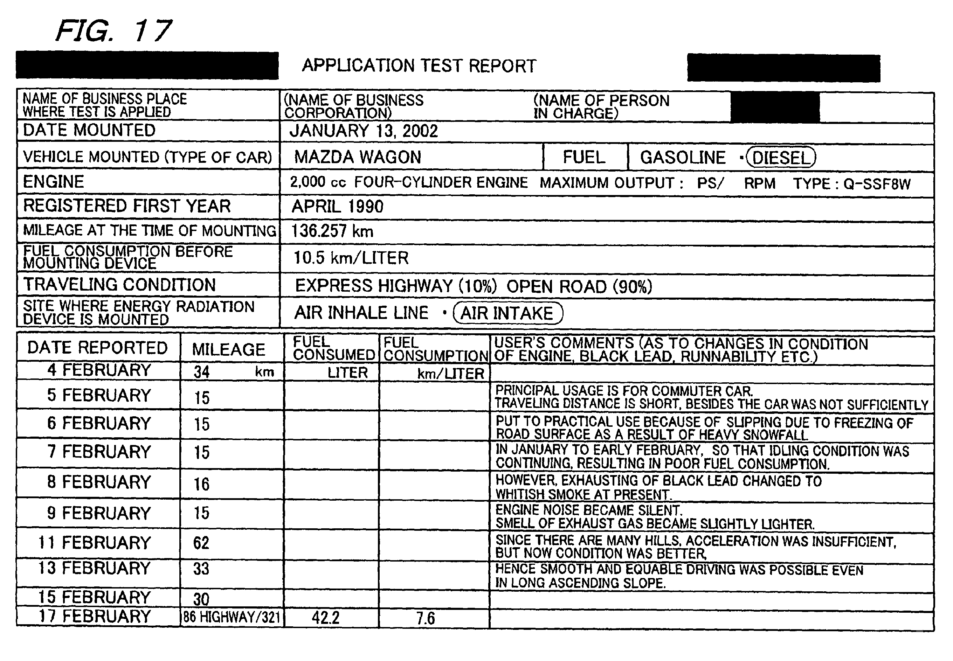

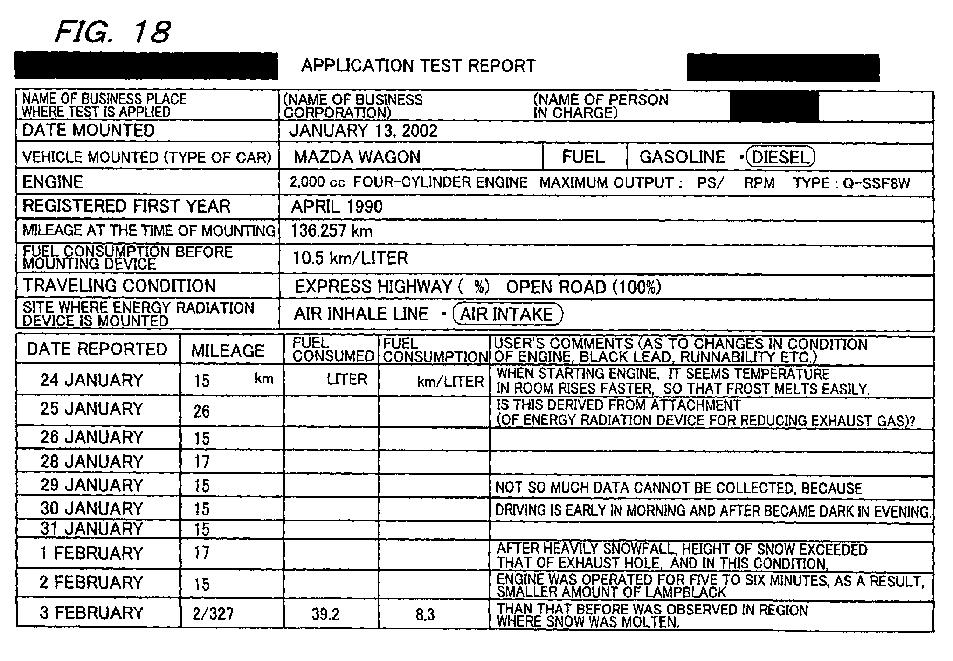

(4) As shown in FIG. 12, when the energy radiation device 10 is mounted on an intake air line 104 positioned between a motor car engine 100 and an air cleaner 102, and color energy E radiated from the energy radiation device 10 is irradiated onto air sucked into the motor car engine 100, a combustion speed in the engine 100 increases. Noₓ, PM and the like in an exhaust gas from the engine are reduced remarkably, whereby fuel consumption is also elevated. In FIGS. 13 to 18, reports of driving experiments as to motor cars wherein the energy radiation device 10 is mounted on the intake air line 104 are indicated. From these reports, it is found that when the energy radiation device 10 is mounted on the intake air line 104, Noₓ, PM and the like in an exhaust gas from the engine are reduced remarkably, whereby fuel consumption is also elevated.

It is preferred to mount the energy radiation device 10 in the vicinity of the engine 100 in the intake air line 104.

A reference character 106 in FIG. 12 is a reflection plate made of an aluminum thin plate or the like, by which it becomes possible to irradiate onto the intake air line 104 without leaking the color energy outside the intake air line 104 by reflecting the color energy E radiated from the energy radiation device 10.

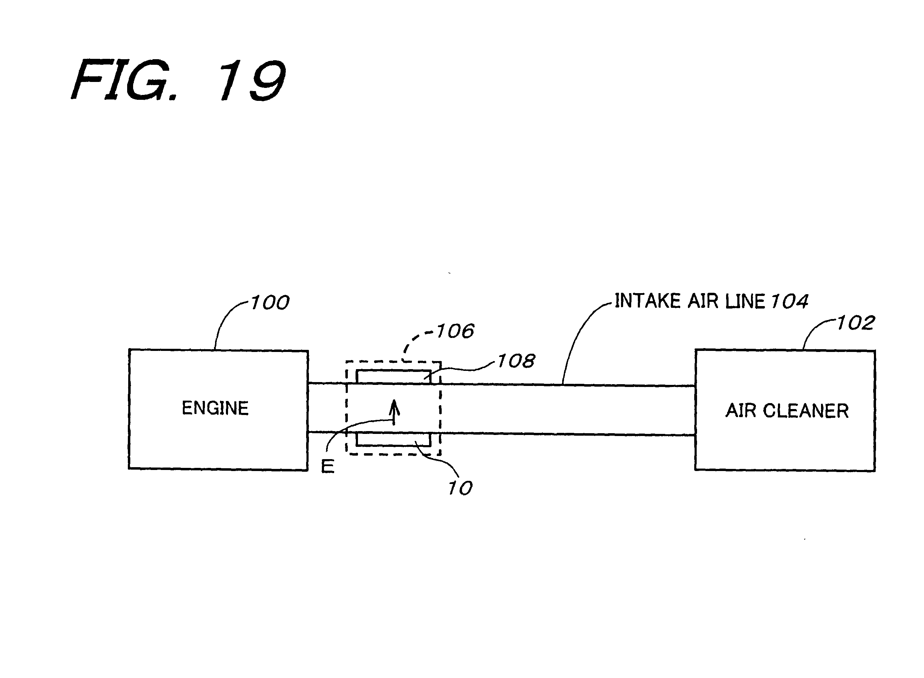

In the case where the material designated by reference character 106 is constituted only in the form of a dustproof cover, but not a reflection plate, for example, a reflection plate 108 made of an aluminum thin plate and the like may be mounted on the intake air line 104 so as to oppose to the energy radiation device 10 as shown in FIG. 19. Hence, it becomes possible that the color energy E radiated from the energy radiation device 10 is irradiated onto the intake air line 104 by means of the reflection plate 108 without leaking the color energy E outside the intake air line 104.

(5) When color energy radiated from the energy radiation device 10 is irradiated onto a smelly substance, it is possible to cancel an appearance of smell, so that it exhibits effects of deodorization or removal of odor.

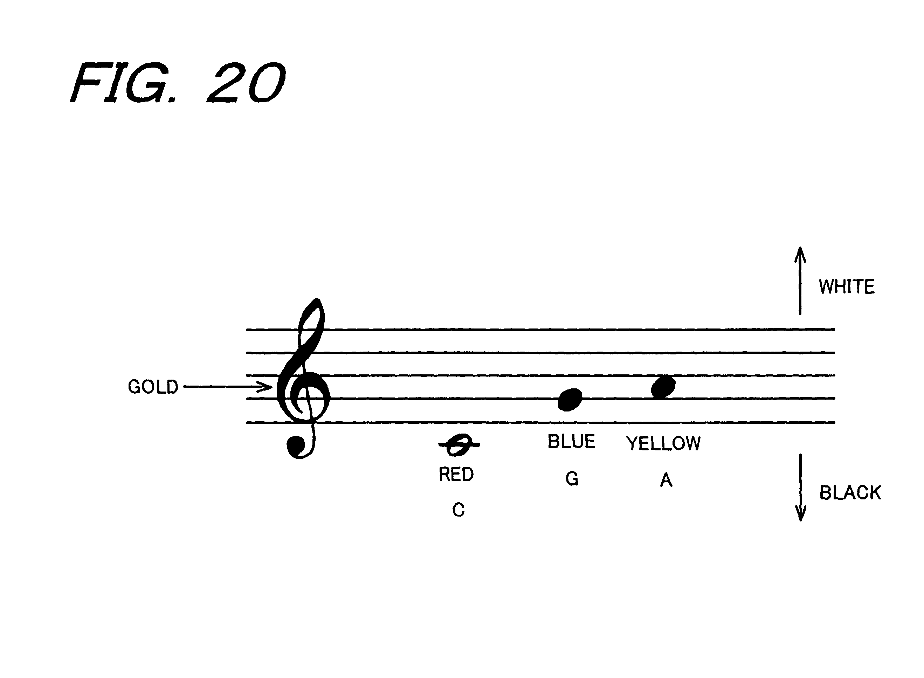

(6) When color energy radiated from the energy radiation device 10 is irradiated onto a musical instrument or a space wherein music is played, harmony intervals which cannot be usually heard or sounds of respective sound pitches are activated, so that functions of five senses in human being who is in touch with the activated sounds or life harmony in plants are maintained. For instance, as shown in harmony melody of sounds in accordance with color energy of FIG. 20, harmony intervals in a musical composition indicated by treble clef in staff notation are activated in the case where a color of the colored material layer 14 in the energy radiation device 10 is gold. When a color of the colored material layer 14 in the energy radiation device 10 is red, a sound having sound pitch of "C" on the lower first added line in staff notation is activated. When a color of the colored material layer 14 in the energy radiation device 10 is blue, a sound having sound pitch of "G" on the second line in staff notation is activated. Furthermore, when a color of the colored material layer 14 in the energy radiation device 10 is yellow, a sound having sound pitch of "A" in the second space in staff notation is activated. When a color of the colored material layer 14 in the energy radiation device 10 is white, sounds having higher sound pitches than that of "A" in the second space in staff notation are activated. Moreover, when a color of the colored material layer 14 in the energy radiation device 10 is black, sounds having higher sound pitches than that of "C" on the lower first added line in staff notation are activated.

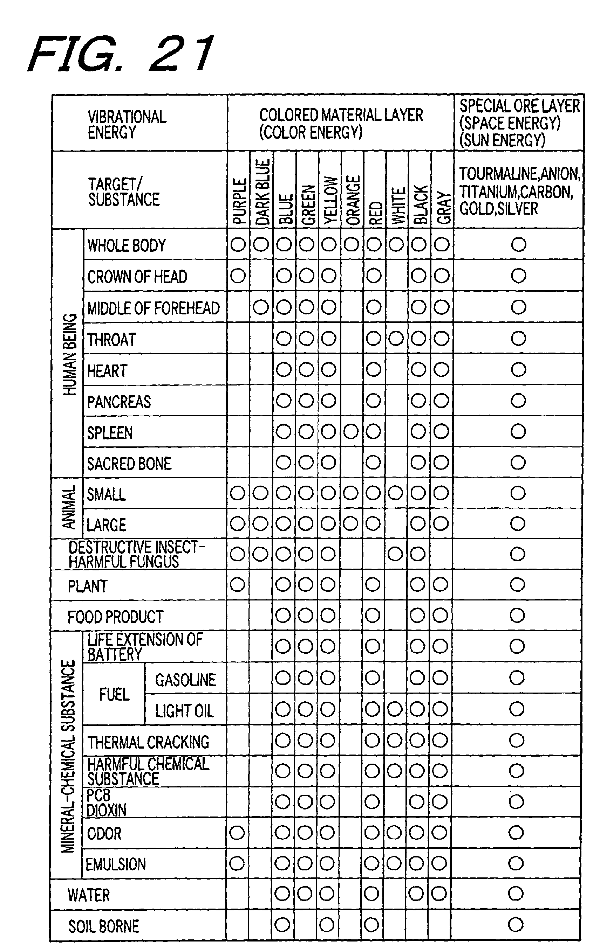

FIG. 21 is a diagram showing a table of effects in materials used in every targets of color energy wherein relationships between colored material layers 16 and special ore layers 14 suitable for acting color energy on respective irradiation targets, in other words, suitable for activating the respective irradiation targets in every color energy irradiation targets are shown.

In the following, an example of an embodiment of an energy irradiation apparatus comprising an energy radiation device according to the present invention will be described in detail by referring to the accompanying drawings.

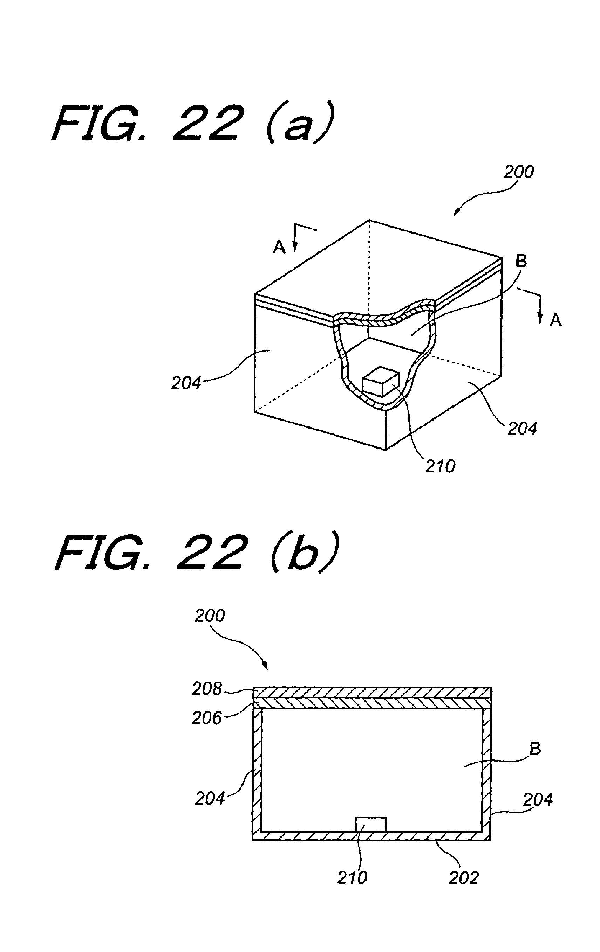

In FIGS. 22(a) and (b) , the energy irradiation apparatus comprising the radiation device according to an example of an embodiment (hereinafter referred optionally to "energy irradiation apparatus" 200 is shown wherein FIG. 22(a) is a partly cutaway perspective view, and FIG. 22 (b) is a sectional view taken along the line A-A of FIG. 22(a).

The energy irradiation apparatus 200 is a hollow box-shaped body the contour of which is substantially rectangularly parallelepipedal shape, and a bottom 202 and four side walls 204 are composed of the energy radiation devices 10. In the case when the energy irradiation apparatus is constituted by the bottom 204 and the four side walls 204 of the energy radiation devices 10, these energy radiation devices are disposed in such that the respective colored material layers 16 are faced to sides of the interior B in the energy irradiation apparatus 200.

Furthermore, a side of the top of the energy irradiation apparatus 200 is shielded with a copper sheet 206, and the top of the copper sheet 206, i.e. an outer side thereof is covered with an aluminum sheet 208.

Under the circumstances, the copper sheet 206 functions to absorb color energy, and the aluminum sheet functions to reflect color energy.

In the interior B of the energy irradiation apparatus 200, a space energy radiation device 210 is disposed on the bottom 202.

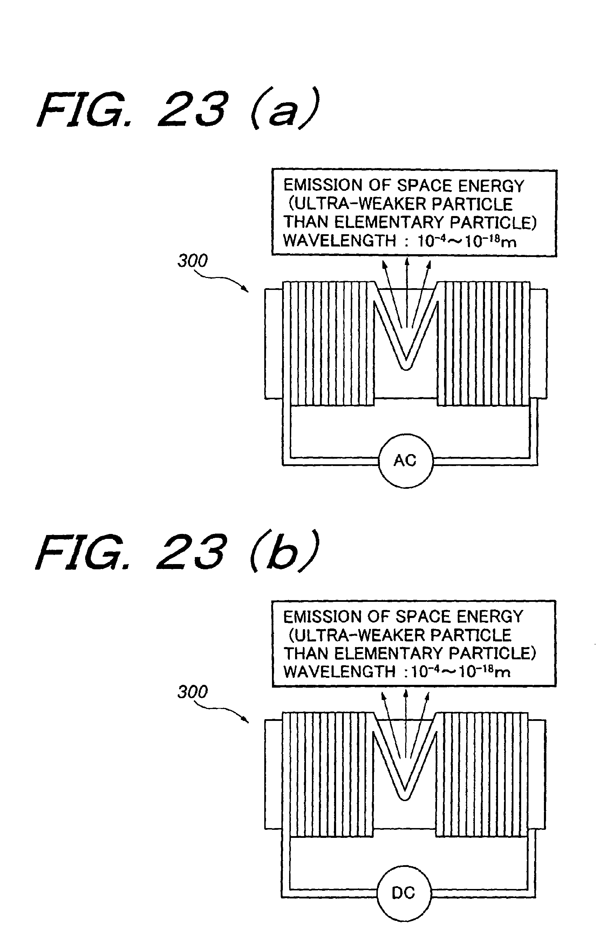

The space energy radiation device 210 may be composed of, for example, a symmetrical reverse winding coil 300 shown in FIG. 23 (a) or FIG. 23 (b) , and space energy may be generated from the symmetrical reverse winding coil 300 to emit the same.

The space energy can be generated to emit the same without producing magnetic field and electromagnetic waves by either applying AC to the symmetrical reverse winding coil 300 as shown in FIG. 23 (a) , or applying DC to the symmetrical reverse winding coil 300 as shown in FIG. 23 (b). The space energy emitted from the symmetrical reverse winding coil 300 is in the form of ultra-fine particles finer than elementary particle, and the space energy has a wavelength of 10⁻⁴ to 10⁻¹⁸ m.

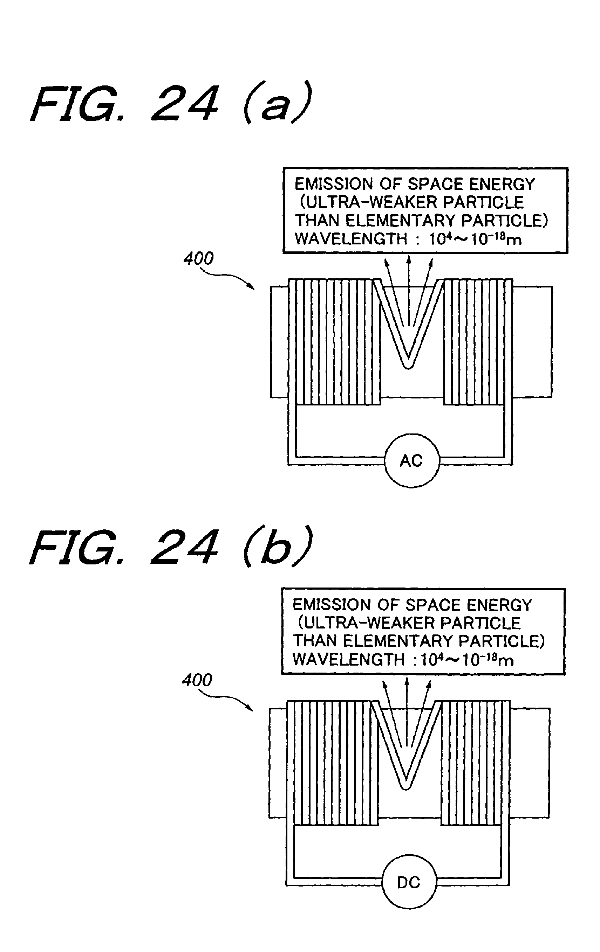

Moreover, the space energy radiation device 210 may also be constituted by, for example, an asymmetrical reverse winding coil 400 as shown in FIG. 24 (a) or FIG. 24 (b) , and space energy can be generated from the asymmetrical reverse winding coil 400 to emit the same.

The space energy can be generated to emit the same without producing magnetic field and electromagnetic waves by either applying AC to the asymmetrical reverse winding coil 400 as shown in FIG. 24 (a) , or applying DC to the asymmetrical reverse winding coil 400 as shown in FIG. 24 (b). The space energy emitted from the asymmetrical reverse winding coil 400 is in the form of ultra-fine particles finer than elementary particle, and the space energy has a wavelength of 10⁻⁴ to 10⁻¹⁸ m.

In the asymmetrical reverse winding coil 400, when the winding number in the symmetrical coil is changed, a wavelength of space energy can be changed.

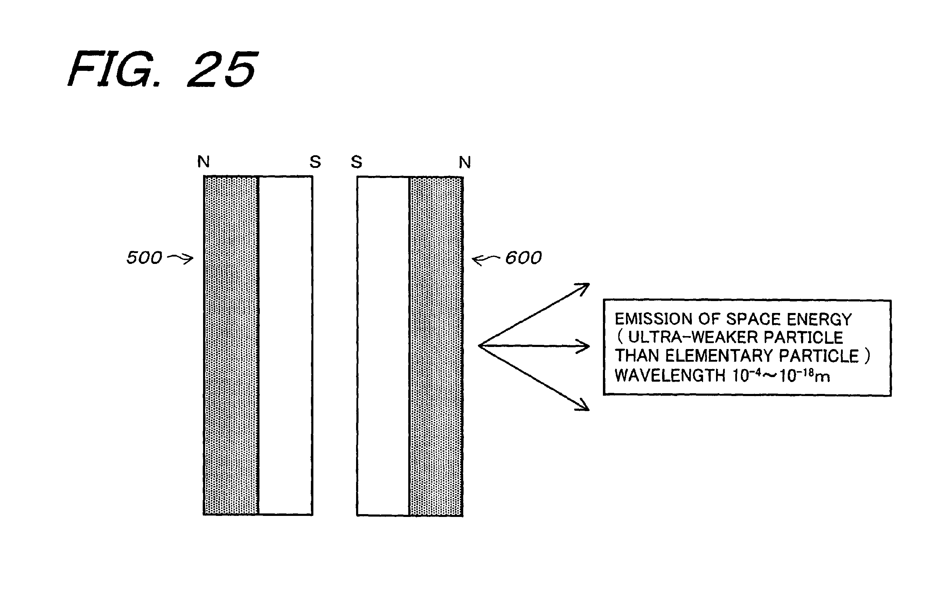

In addition, the space energy radiation device 210 may be disposed, for example, in its construction so as an NS magnet 500 to repel an SN magnet 600 as shown in FIG. 25, whereby generation of magnetic field and electromagnetic wave is suppressed to produce space energy to emit the same.

In case of the construction shown in FIG. 25, the space energy emitted is in the form of ultra-fine particles finer than elementary particle, and the space energy has a wavelength of 10⁻⁴ to 10⁻¹⁸ m.

In the above-described construction, when any of the whole targets existing on the earth such as water in a container is placed in the interior B of the energy irradiation apparatus 200 and space energy is emitted from the space energy radiation device 210, color energy is irradiated on the interior B of the energy radiation device 200 in accordance with the functions as described above, so that the color energy acts on the target placed in the interior B of the energy irradiation apparatus 200.

In this case, the copper sheet 206 absorbs color energy, while the aluminum sheet 208 reflects color energy, so that such color energy is efficiently irradiated onto the target placed in the interior B of the energy irradiation apparatus 200, whereby the target can be promptly activated.

It is to be noted that when colors of colored material layers of the energy radiation device 10 constituting the bottom 202 and four side walls 204 are properly combined with each other, the energy irradiation apparatus 200 may be used in a variety of applications.

The above-described embodiment may be modified into those shown in the following paragraphs (1) through (8).

### (1) First Modification

In the above-described embodiment, although the special ore layer 14 is laminated on the substrate 12, and further the colored material layer 16 is laminated thereon, the invention is not limited thereto, as a matter of course, but the colored material layer 16 may be simply laminated on the special ore layer 14 without using the substrate 12. After all, it is sufficient that the special ore layer is laminated with the colored material layer in the energy radiation device according to the present invention.

### (2) Second Modification

In the above-described embodiment, although the special ore layer 14 is laminated on the whole surface of the substrate 12, besides the colored material layer 16 is laminated on the whole surface of the special ore layer 14, the invention is not limited thereto, as a matter of course, but it is sufficient that the special ore layer 14 may be laminated only on a part of the top of the substrate 12, and the colored material layer 16 may be laminated only on a part of the top of the special ore layer 14. After all, it is sufficient that the special ore layer is laminated with even a part of the colored material layer in their regions in the energy radiation device according to the present invention.

### (3) Third Embodiment

In the above-described embodiment, although the special ore layer 14 is laminated on the substrate 12, besides only a single layer of the colored material layer 16 is laminated thereon, the invention is not limited thereto, as a matter of course. However, the invention may be, for example, constituted, as shown in FIG. 4(b), in such that plural layers of the colored material layers 16 made of colored materials having the same colors may be laminated. Furthermore, as shown in FIG. 4(c), the invention may be constituted in such that plural layers of the colored material layers 16 made of colored materials 16, 16', 16", and 16'" having different colors one another may be laminated.

### (4) Fourth Modification

In the above-described embodiment and the above-described third modification, although the colored material layer 16 is directly laminated on the special ore layer 14 (see FIG. 4 (a) ) , the colored material layer 16 is directly laminated on the other colored material layer 16 (see FIG. 4(b)), or plural layers of the colored material layers 16, 16', 16", and 16'" having different colors one another are directly laminated, respectively (see FIG. 4(c)). However, the invention is not limited thereto, as a matter of course. For instance, it may be arranged in such that an intermediate layer 18 containing gold, silver, titanium, carbon or the like is laminated on the special ore layer 14, and the colored material layer 16 is laminated on the resulting intermediate layer 18 (see FIG. 5 (a) corresponding to FIG. 4(a)), that an intermediate layer 18 containing gold, titanium or the like is laminated on the colored material layer 16, and the other colored material layer is laminated on the resulting intermediate layer 18 (see FIG. 5(b) corresponding to FIG. 4(b)), and that each intermediate layer 18 containing gold, silver, titanium, carbon or the like is sandwiched between each pair of plural layers of the colored material layers 16, 16', 16", and 16'" having different colors one another to be laminated.

In this case, the intermediate layer 18 containing gold, silver, titanium, carbon or the like may be composed of, for example, gold or silver colored paper, or it may be constituted in such that a very little amount of gold foil, silver foil, titanium pieces or carbon pieces is enveloped by a white paper.

As described above, color energy radiated from an energy radiation device according to the present invention is amplified by a provision of the intermediate layer 18 containing gold, silver, titanium, carbon or the like.

It is further to be noted that in the modification shown in FIG. 5(b), an intermediate layer 18 containing gold, silver, titanium, carbon or the like is not necessarily required to be inserted into each pair of respective colored material layers 16, but only a single intermediate layer 18 may be provided between any pair of the colored material layers 16. Similarly, each intermediate layer 18 containing gold, silver, titanium, carbon or the like is not necessarily required to be inserted into each pair of respective colored material layers 16, 16', 16" and 16'", but only a single intermediate layer 18 may be provided, for example, between the colored material layers 16' and 16".

### (5) Fifth Modification

In the above-described embodiment, although the energy irradiation apparatus 200 is constituted into a hollow box-shaped body the contour of which is a rectangularly parallelepipedal shape, the invention is not limited thereto as a matter of course. For instance, the energy irradiation apparatus may be constituted in a hollow body the contour of which is a cubical shape, or a hollow body the contour of which is a spherical shape. After all, it is sufficient to form a space (a sealed space is preferable) defined by a wall or walls of the energy irradiation apparatus 10, and to dispose a space energy radiation device in the space. Furthermore, either all the wall surfaces of the energy irradiation apparatus 200 may be composed of the energy radiation devices 10, or a part of the energy irradiation apparatus 200 may be composed of the energy radiation device (or devices) 10.

### (6) Sixth Modification

In the above-described embodiment, although only one space energy radiation device 210 is disposed in the interior B of the energy irradiation apparatus 200, both cases of a single space energy radiation device 210 and plural space energy radiation devices 210 may be permitted in the present invention.

### (7) Seventh Modification

In the above-described embodiment, although the copper sheet 206 and the aluminum sheet 208 are placed on the upper side of the energy irradiation apparatus 200, the invention is not limited thereto as a matter of course. For instance, only either of the copper sheet 206 and the aluminum sheet 208 may be provided, or both the sheets may not be provided. In case of providing only the copper sheet 206, it is preferred to dispose the energy radiation device 10 on the upper side of the copper sheet 206. Moreover, in case of no provision of any of the copper sheet 206 and the aluminum sheet 208, it is preferred to dispose the energy radiation device 10 in place of both the sheets.

(8) The above-described embodiment may be optionally combined with the first through the seventh modifications described in the above paragraphs (1) through (7).

### Industrial Applicability

Since the present invention has been constituted as described above, there is such an excellent advantage to provide an energy radiation device by which benefits of tourmaline can be efficiently and easily utilized, besides to provide an energy irradiation apparatus comprising the same.

## Claims

1. An energy radiation device, comprising:
a special ore layer prepared by mixing tourmaline with an anion ore; and
a colored material layer made of a colored material having a predetermined color and which is laminated on said special ore layer.

2. An energy radiation device, comprising:
a special ore layer prepared by mixing tourmaline with an anion ore; and
plural colored material layers each made of a colored material having a predetermined color and which are laminated on said special ore layer.

3. The energy radiation device as claimed in claim 2, wherein:
said plural colored material layers are prepared from colored materials having different colors one another.

4. The energy radiation device as claimed in any one of claims 1, 2, and 3, wherein:
an intermediate layer containing gold, silver, titanium or carbon is provided between said special ore layer and said colored material layer.

5. The energy radiation device as claimed in any one of claims 2, 3, and 4, wherein:
an intermediate layer containing gold, silver, titanium or carbon is provided between any one of regions defined between said plural colored material layers.

6. An energy irradiation apparatus provided with the energy radiation device as claimed in any one of claims 1, 2, 3, 4, and 5, comprising:
a hollow body a part of or the whole walls of which are constituted by said energy radiation device or devices; and
a space energy radiation device disposed in the interior of said hollow body.

7. The energy irradiation apparatus provided with the energy radiation device as claimed in claim 6, wherein:
a copper sheet is disposed on a part of said walls.

8. The energy irradiation apparatus provided with the energy radiation device as claimed in any one of claims 6 and 7, wherein:
an aluminum sheet is disposed on a part of said walls.

9. The energy irradiation apparatus provided with the energy radiation device as claimed in 7, wherein:
the outer side of said copper sheet is covered with an aluminum sheet.

10. The energy irradiation apparatus provided with the energy radiation device as claimed in any one of claims 6, 7, 8, and 9, wherein:
said space energy radiation device is a symmetrical reverse winding coil.

11. The energy irradiation apparatus provided with the energy radiation device as claimed in any one of claims 6, 7, 8, and 9, wherein:
said space energy radiation device is an asymmetrical reverse winding coil.

12. The energy irradiation apparatus provided with the energy radiation device as claimed in any one of claims 6, 7, 8, and 9, wherein:
said space energy radiation device is constituted so as an NS magnet to repel an SN magnet.
